**Europäisches Patentamt**

**European Patent Office**

**Office européen des brevets**

(19)

(11) Veröffentlichungsnummer : **0 240 034 B1**

(12)

# EUROPÄISCHE PATENTSCHRIFT

(45) Veröffentlichungstag der Patentschrift :
**15.01.92 Patentblatt 92/03**

(51) Int. Cl.⁵ : **A61B 17/60**

(21) Anmeldenummer : **87105092.8**

(22) Anmeldetag : **06.04.87**

(54) Fixateur externe zur Osteosynthese.

(30) Priorität : **04.04.86 DE 3611319**

(43) Veröffentlichungstag der Anmeldung :
**07.10.87 Patentblatt 87/41**

(45) Bekanntmachung des Hinweises auf die
Patenterteilung :
**15.01.92 Patentblatt 92/03**

(84) Benannte Vertragsstaaten :
**AT BE CH DE ES FR GB GR IT LI LU NL SE**

(56) Entgegenhaltungen :
**EP-A- 0 140 786**
**DE-A- 2 929 455**
**FR-A- 782 462**
**FR-A- 2 499 400**
**FR-A- 2 531 332**
**GB-A- 2 031 731**

(56) Entgegenhaltungen :
**GB-A- 2 157 179**
**"Current Concepts of External Fixation of**
**Fractures" herausgegeben von Uhthoff und**
**Stahl, erschienen beim Springer-Verlag Berlin**
**Heidelberg New York 1982, Seite 99-103**

(73) Patentinhaber : **Witzel, Ulrich, Dr.-Ing.**
**Wittener Strasse 73d**
**W-5600 Wuppertal 2 (DE)**

(72) Erfinder : **Witzel, Ulrich, Dr.-Ing.**
**Wittener Strasse 73d**
**W-5600 Wuppertal 2 (DE)**
Erfinder : **Müller, Karl, H. Prof.Dr.med.**
**Unterfeldstrasse 27**
**W-4630 Bochum (DE)**

(74) Vertreter : **Ostriga, Harald et al**
**Stresemannstrasse 6-8 Postfach 20 13 27**
**W-5600 Wuppertal 2 (DE)**

## Beschreibung

Die Erfindung betrifft einen Fixateur externe zur Osteosynthese, wie er im Oberbegriff des Patentanspruchs 1 beschrieben und in dem Buch "Current Concepts of External Fixation of Fractures", herausgegeben von Uhthoff und Stahl, erschienen beim Springer-Verlag Berlin Heidelberg New York 1982, s. dort 99-103, vorbekannt ist.

Als wesentliche Elemente enthält der vorbekannte Fixateur zwei etwa kreiszylindrische Stahlscheiben gleichen Umfangs, welche von einer gemeinsamen Klemmschraube koaxial durchgriffen sind. In der Fuge zwischen den beiden Klemmscheiben kann gegebenenfalls eine dünne Reibscheibe vorgesehen sein. Als Klemmbacken zur Aufnahme des Überbrückungsbauteils (Fixateurstange) weist eine Klemmscheibe des Klemmscheibenpaares zwei sich sekantial und parallel zueinander erstreckende etwa zylindrische Bohrungen auf, welche die der benachbarten Klemmscheibe zugewandte radiale Kreisstirnfläche durchdringen. Es ergeben sich so zwei über einen relativ großen Umfangsbereich längsgeschlitzte Aufnahmebohrungen, in welche die Fixateurstangen - aus den Längsschlitzen einseitig längsseits vorragend - axial eingeschoben werden müssen.

Die zylindrischen Halteöffnungen zur Aufnahme entweder eines Knochennagels (z.B. eines Steinmann-Nagels) beziehungsweise einer Knochenschraube (z.B. einer Schanze'schen Schraube) sind beim vorbekannten Fixateur analog zu den beschriebenen zylindrischen Bohrungen für die Fixateurstangen ebenfalls als einseitig längsgeschlitzte zylindrische Bohrungen ausgebildet.

Eine Verklemmung einer Fixateurstange und einer Knochenschraube geschieht bei dem vorbekannten Fixateur externe demnach gemeinsam durch Anzug der zentralen Klemmschraube derart, daß beide Klemmscheiben mit ihren zugewandten Kreisstirnflächen axial gegeneinandergepreßt werden und hierbei die aus den Längsschlitzen der Aufnahmebohrungen vorstehenden Umfangsbereiche von Fixateurstange und Knochenschraube zwischen sich verklemmen.

Der durch das Buch "Current Concepts of External Fixation of Fractures" vorbekannte Fixateur externe bietet im Unterschied zu anderen durch offenkundige Vorbenutzung vorbekannten Fixateuren bereits den Vorteil einer nur wenige Bauteile umfassenden einfachen Bauform, ist jedoch hinsichtlich seiner Handhabung während der Operation und der nachherigen Versorgung des Patienten verbesserungsbedürftig.

Ausgehend von dem aaO in dem Buch "Current Concepts of External Fixation of Fractures" beschriebenen Fixateur externe, liegt der Erfindung die Aufgabe zugrunde, den vorbekannten Fixateur externe unter Beibehaltung seiner vorteilhaften einfachen Bauform handhabungstechnisch zu verbessern. Diese Aufgabe wird entsprechend dem Kennzeichenteil des Anspruchs 1 gelöst.

Im Unterschied zum vorbezeichneten bekannten Fixateur externe (s. "Current Concepts ..."), ist jede zylindrische Halteöffnung von einer einteiligen zusammenhängenden Laibungsfläche gebildet, welche durch Betätigung eines Schraubelements elastisch oder plastisch mit einem derartigen Ausmaß verformbar ist, daß die zylindrische Halteöffnung zunächst führend als Bohr- oder Schraublehre und - nach fortschreitender Zustellung bzw. Verengung - als Klemmaufnahme für den Knochennagel bzw. für die Knochenschraube dient. Entsprechend der Erfindung ist es grundsätzlich möglich, die zylindrischen Halteöffnungen gemeinsam mit benachbarten Klemmbackenflächen, die der Aufnahme des Überbrückungsbauteils (insbesondere Fixateurstange) dienen, zuzustellen.

Eine bevorzugte Ausgestaltung der Erfindung besteht darin, daß die zylindrischen Halteöffnungen bei Anzug der Betätigungsschrauben durch elastische oder plastische Werkstoffverformung bezüglich des Überbrückungsbauteils zunächst im Sinne einer Vorarretierung zustellbar und jeweils mit einem zusätzlichen Zustellhub von einer Bohrer- bzw. Schraubenführung bis zu ihrer Verklemmungs-Endlage mit dem Knochennagel bzw. mit der Knochenschraube verformbar sind.

Eine bevorzugte erfindungsgemäße Ausführungsform kann deshalb in der Praxis mit Vorteil wie folgt gehandhabt werden: eine zylindrische Halteöffnung wird - gegebenenfalls gemeinsam mit benachbarten Klemmbackenflächen - durch Teilanzug der Betätigungsschraube vorarretiert. Dies bedeutet, daß die Halterung auf dem Überbrückungsbauteil (z.B. auf einer Fixateurstange) im wesentlichen unverschieblich gehalten und die zylindrische Halteöffnung bezüglich des Überbrückungsbauteils bzw. bezüglich der sie tragenden Halterung im wesentlichen unverstellbar positioniert ist. Durch die zylindrische Halteöffnung hindurch können sodann zum Beispiel ein Knochenbohrer (nachfolgend gegebenenfalls ein Gewindebohrer) und, nach Herausnahme des Knochen- öder Gewindebohrers, eine Knochenschraube bzw. ein Knochennagel (letztere als Implantat) eingesetzt werden. Sobald der Knochennagel bzw. die Knochenschraube durch die zylindrische Halteöffnung hindurch befestigt ist und sich in ihrer Endlage innerhalb des Knochens befindet, erfolgt die restliche Zustellung der zylindrischen Halteöffnung bis zur Verklemmung.

Aus vorstehender Schilderung wird deutlich, daß der erfindungsgemäße Fixateur operationstechnisch eine wesentliche Vereinfachung gegenüber dem Bekannten darstellt.

Entsprechend der Erfindung ist ein Fixateur externe dann besonders vorteilhaft, wenn jeweils mit einer einzigen Betätigungsschraube sowohl die zylindrische Halteöffnung (zur sukzessiven Aufnahme eines Bohrers und einer Knochenschraube oder eines Knochennagels) als auch die Klemmbackenflächen der zugeordneten Halterung, und zwar von der Vorarretierung bis zur Endlagenarretierung, bedient werden können. Zu einer Vereinfachung der Bedienung können hierbei die Betätigungsschrauben als mit manuell bedienbaren großen Handhabungsflächen versehene Knebelschrauben ausgebildet sein. Eine elastische oder plastische Verformung der zylindrischen Halteöffnung - fakultativ gemeinsam mit benachbarten Klemmbackenflächen für das Überbrückungsbaueil - läßt sich dann vorteilhaft durchführen, wenn die an der Zustellung beteiligten Bauteile aus einem geeigneten Kunststoff bestehen. Ein solcher Kunststoff kann zu einem Kunststoffspritzgußteil verarbeitet und insbesondere kohlefaser- oder glasfaserverstärkt sein. Eine Verwendung von Kunststoffen - gegebenenfalls unter Einbeziehung der Überbrückungsbauteile - schafft neben einer Vereinfachung und Verbilligung der Herstellung und einer leichten Bauweise eine erstrebenswerte Röntgenstrahlen-Transparenz, die bei den bislang gebräuchlichen Metall-Fixateuren nicht gegeben ist.

Weitere Vorteile entsprechend der Erfindung ergeben sich aus zusätzlichen Unteransprüchen.

In den Zeichnungen sind bevorzugte Ausführungsbeispiele der Erfindung näher dargestellt, es zeigen

Fig. 1 einen externen lateralen Klammer- oder Monofixateur, dargestellt in Verbindung mit einer an der Tibia vorzunehmenden Distanzosteosynthese;

Fig. 2 eine Ausführungsform mit einer zweiteiligen Halterung, deren eines Bauteil der Aufnahme einer Knochenschraube und deren anderes Bauteil der Aufnahme einer Fixateurstange dient;

Fig. 3 eine Schnittdarstellung entsprechend der mit III bezeichneten Schnittlinie in Fig. 2;

Fig. 4 eine Schnittdarstellung entsprechend der Schnittlinie IV-IV in Fig. 3;

Fig. 5 eine Ausführungsform, gemäß welcher zwei Fixateur-Haltestangen zusammenwirken;

Fig. 6 eine Einzeldarstellung ohne Betätigungsschraube und ohne Fixateurstange der unteren Teilhalterung gemäß Fig. 5;

Fig. 7 eine Schnittdarstellung entsprechend der Schnittlinie VII-VII in Fig. 6;

Fig. 8 eine mit einer Klemmschelle versehene andere Ausführungsform, bei welcher unmittelbar über eine einzige Betätigungsschraube sowohl ein Knochennagel als auch eine Fixateurstange arretiert werden können;

Fig. 9 eine Schnittdarstellung gemäß der Schnittlinie IX-IX in Fig. 8;

Fig. 10 eine Einfach-Klemmschelle analog zur Darstellung in Fig. 8, bei welcher jedoch die Verklemmung der Fixateurstange mittelbar erfolgt;

Fig. 11 eine zweigeteilte Doppelklemmschelle, etwa als Doppelanordnung der in Fig. 10 gezeigten Anordnung;

Fig. 12 eine Anordnung analog zu Fig. 11, bei welcher jedoch beide Klemmbackenbereiche der Doppelschelle schlitzrohrförmig ausgebildet sind;

Fig. 13 analog zu Fig. 12 eine Doppelklemmschelle mit einer raumgelenkigen Positionierung und Arretierung einer Knochenschraube und dem Vorteil einer direkten Arretierung der beiden Fixateurstangen durch die Betätigungsschraube;

Fig. 14 und 15 weitere Ausführungsformen analog zu der gemäß Fig. 14;

Fig. 16-19 verschiedene stopfenartige Halterungen zum Einsatz in etwa platten- bzw. wabenförmige Überbrückungsbauteile.

Soweit möglich, sind für gleiche oder ähnliche Teile dieselben oder ähnliche Bezugszeichen verwendet.

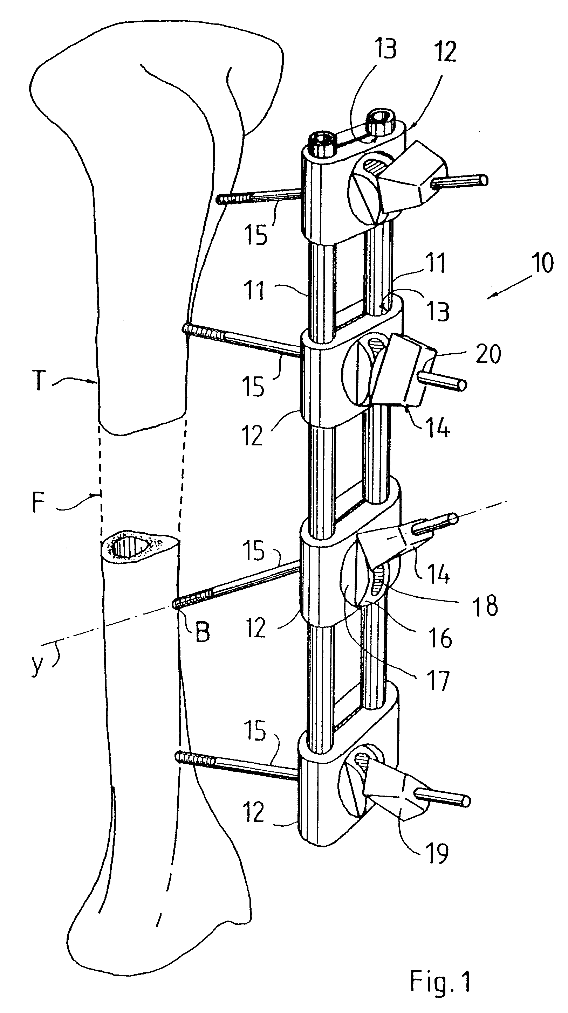

In Fig. 1 ist ein gesamter Fixateur externe mit der Bezugsziffer 10 bezeichnet. Der Fixateur externe ist am Beispiel einer mit T bezeichneten Tibia gezeigt, bei welcher im Frakturbereich F eine Distanzosteosynthese vorgenommen wird.

Hierzu weist der Fixateur 10 zwei Fixateurstangen 11 auf, welche in vier Halterungen 12 klemmarretiert sind. Jede Halterung 12 ist mit Klemmbackenflächen 13 zur Aufnahme der Fixateurstangen 11 versehen.

Die Verklemmung der Klemmbackenflächen 13 auf den Fixateurstangen 11 wird durch Betätigungsschrauben 14 bewirkt. Durch Anzug der Betätigungsschrauben 14 werden zugleich aus Fig. 1 nicht entnehmbare zylindrische Halteöffnungen bis zur Verklemmung mit den in ihnen aufgenommenen Knochenschrauben 15 (sogenannte "Schanze'sche Schrauben") zugestellt.

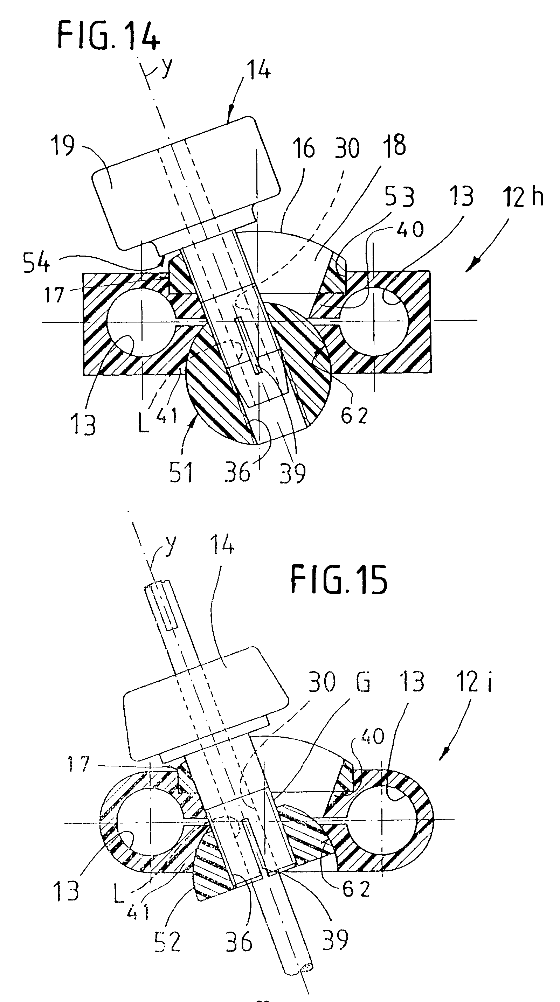

Zur Aufnahme der Knochenschrauben 15 sind Bauteile vorgesehen, wie sie etwa in den Fig. 14 bzw. 15 gezeigt sind. Zu diesen Bauteilen gehören Unterlegscheiben 17, die der räumlichen Ausrichtung der Knochenschrauben 15 dienen. Die drehbaren Unterlegscheiben 17 sind im Bereich ihrer balligen Oberfläche 16 mit einem Langloch 18 zur Aufnahme des nicht gezeigten Schraubenschaftes der Betätigungsschraube 14 versehen, die wiederum die Knochenschraube 15 enthält.

Die praktische Handhabung des in Fig. 1 dargestellten Fixateurs externe vollzieht sich etwa wie folgt:

Die Relativlage der Halterungen 12 auf den Überbrückungsbauteilen (Fixateurstangen 11) wird durch teilweisen Anzug der betreffenden Betätigungsschraube 14 mit deren Handbetätigungsknebel 19 bewirkt. Durch diesen Teilanzug wird außerdem die Längsachse y jeder Betätigungsschraube in einer gewünschten räumlichen Lage vorarretiert.

Durch die stirnseitige Öffnung 20 (die in die nicht gezeigte zylindrische Halteöffnung übergeht) der betreffenden Betätigungsschraube 14 wird sodann in nicht gezeigter Weise ein Knochenbohrer axial hindurchgeführt und die Haltebohrung B in die Tibia eingebracht. Nach Herausnahme des Knochenbohrers wird sodann durch die Öffnung 20 der Betätigungsschraube 14 hindurch ein Gewindebohrer eingesetzt und die Knochenbohrung B mit einem Innengewinde versehen. Nach Herausdrehen des Knochen-Gewindebohrers wird sodann durch die Öffnung 20 der Betätigungsschraube 14 hindurch die Knochenschraube 15 eingesetzt. Nachdem die Knochenschraube 15 ihre endgültige Befestigungslage in der Knochenbohrung B erreicht hat, wird die betreffende Betätigungsschraube 14 mittels des Betätigungsknebels 19 manuell zugrundegezogen. Hierdurch werden sowohl die Halterung 12 auf der zugeordneten Fixateurstange 11 als auch die Knochenschraube 15 innerhalb der Betätigungsschraube 14 und auch schließlich das Raumgelenksystem (von dem nur die Unterlegscheibe 17 in Fig. 1 gezeigt ist) relativ zur Fixateurstange 11 fest klemmarretiert.

Anhand der Darstellung gemäß Fig. 1 ist ohne weiteres vorstellbar, daß die dort gezeigten Bauelemente auch zur Erstellung eines Rahmenfixateurs oder eines Fixateurs nach Art eines Raumfachwerks verwendet werden können. Hierbei würden nicht dargestellte zusätzliche Halterungen 12 einseitig an den beiden Fixateurstangen 11 angeklemmt sein und als Kupplungsglieder zu weiteren nicht dargestellten seitlich benachbarten Fixateurstangen 11 dienen. Auf diese Weise können auch zur Halterung von Steinmann-Nägeln Halterungen 12 (mit Betätigungsschrauben 14) an einander diametral gegenüberliegenden Seiten der Tibia T angeordnet werden. Die Verwendung des Fixateurs 10 ist selbstverständlich nicht auf eine Tibia T beschränkt sondern vorteilhaft auch bei anderen Extremitäten sowie des Beckens, gegebenenfalls auch gelenkübergreifend, möglich.

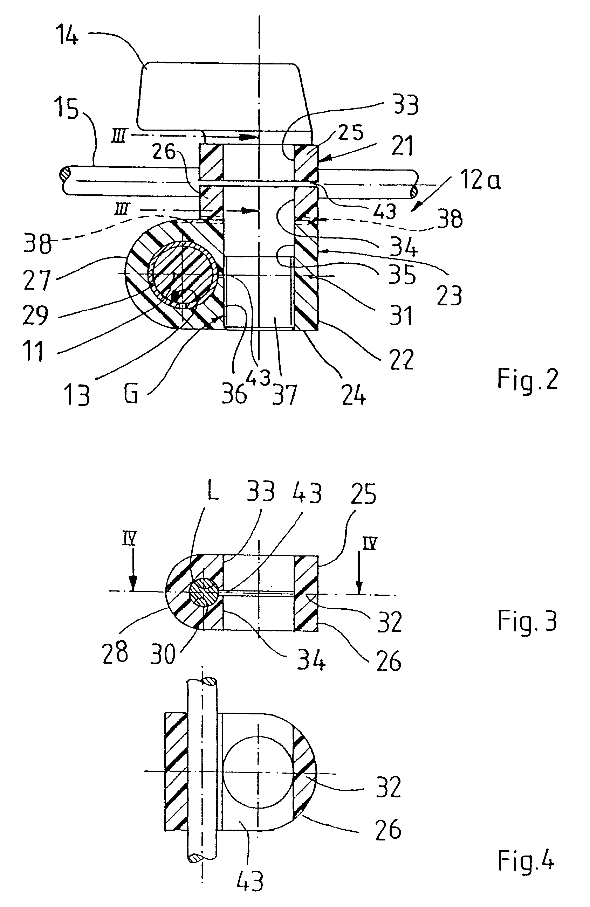

Beim Ausführungsbeispiel gemäß den Fig. 2-4 weist die zweiteilige Halterung 12 a zwei Klemmschellen 21, 22 auf.

Jede Klemmschelle 21, 22 besitzt je zwei einander parallele Klemmschenkel 23, 24 sowie 25, 26. Jeweils zwei Klemmschenkel 23, 24 bzw. 25, 26 sind durch einen Schlitzrohrbereich 27, 28 miteinander verbunden.

Der Schlitzrohrbereich 27 der Klemmschelle 22 bildet die Klemmbackenfläche 13 für die aus kohlefaserverstärktem Kunststoff bestehende Fixateurstange 11. Für den Fall, daß Fixateurstangen 11 mit Durchmessertoleranzen verwendet werden, kann zum Ausgleich eines Fixateurstangen-Untermaßes eine Schlitzhülse 29 in die Klemmbackenfläche 13 eingeschoben werden. Die Schlitzhülse 29 bildet demnach eine umfangsveränderliche Paßbuchse.

Die andere Klemmschelle 21 enthält die zylindrische Halteöffnung 30 zur Aufnahme der Knochenschraube 15.

Eine Besonderheit bei beiden Klemmschellen 21 und 22 besteht darin, daß die jeweils nur einen Schlitzrohrbereich 27 bzw. 28 aufweisenden Klemmschellen 21 und 22 endseitig ihrer beiden Klemmschenkel 23, 24; 25, 26 über je eine Werkstoffbrücke 31, 32 einstückig-stoffschlüssig miteinander verbunden sind. Die Werkstoffbrücken 31, 32 dienen der Erzielung definierter Klemmverhältnisse.

Von den vier aufeinanderfolgenden Klemmschenkeln 25, 26, 23, 24 weisen drei Klemmschenkel 25, 26, 23 je eine glatte Durchgangsbohrung 33, 34, 35 und der eine Klemmschenkel 24 eine Innengewindebohrung 36 für das Gewindeteil 37 der gemeinsamen Betätigungsschraube 14 auf, welche bei der Ausführungsform gemäß den Fig. 2-4 die zylindrische Halteöffnung 30 selbst nicht bildet.

Bei der Ausführungsform gemäß den Fig. 2-4 werden sowohl die Klemmbackenfläche 13 als auch die zylindrische Halteöffnung 30 gemeinsam über die Betätigungsschraube 14 bedient. Wie bereits im Zusammenhang mit Fig. 1 dargelegt, ist hierbei eine Vorarretierung der Halterung 12 a bezüglich der Fixateurstange 11 und der Knochenschraube 15 bzw. bezüglich eines nicht dargestellten Bohrers möglich. Erst ein weiteres Zugrundeziehen der Betätigungsschraube 14 bewirkt eine gemeinsame feste Endlagenverklemmung der Fixateurstange 11 bezüglich der Klemmbackenfläche 13 bzw. bezüglich der Schlitzhülse 29 und der Knochenschraube 15 bezüglich der zylindrischen Halteöffnung 30.

Zur Sicherung der relativen Drehlage der Klemmschellen 21, 22 zueinander ist an den einander zugewandten Berührungsflächen je eine Plan-Kerb-Verzahnung, sogenannte "Hirth-Verzahnung", welche die Bezugsziffer 38 trägt, vorgesehen.

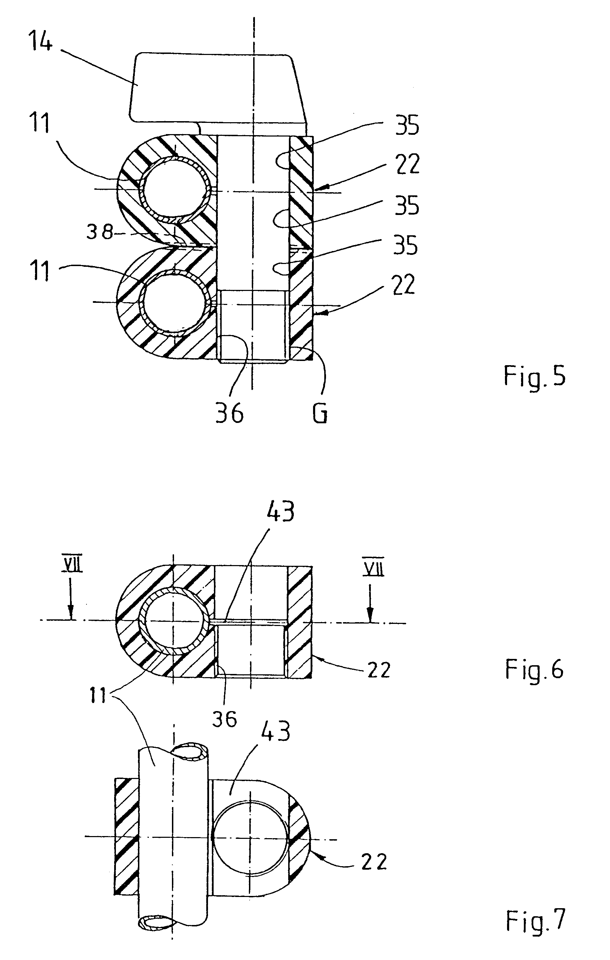

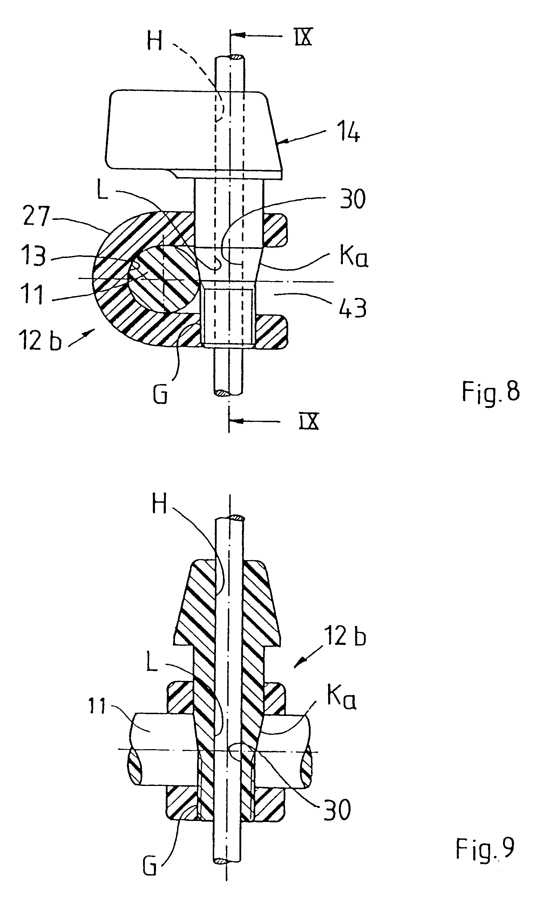

Bei der Ausführungsform gemäß den Fig. 5-7 sind zwei im wesentlichen gleiche Klemmschellen 22, 22 vorgesehen, von denen jedoch die obere zwei glatte Durchgangsbohrungen 35 aufweist, während die untere Klemmschelle 21 eine glatte Durchgangsbohrung 35 und eine Innengewindebohrung 36 besitzt. Die Verklemmung der beiden je eine rohrförmige Fixateurstange 11 aufnehmenden Klemmschellen 22, 22 geschieht analog zum Ausführungsbeispiel gemäß den Fig. 2-4. Eine zylindrische Halteöffnung 30 ist bei der Ausführungsform gemäß den Fig. 5-7 nicht gezeigt, jedoch ist es ohne weiteres denkbar, daß die Betätigungsschraube 14 eine zylindrische Halteöffnung 30, etwa analog zur Betätigungsschraube 14 gemäß Fig. 8, enthält.

Bei der Ausführungsform gemäß den Fig. 8 und 9 ist die Betätigungsschraube 14 von einem Axialhohlraum H durchsetzt, welcher die Laibungsfläche L zur Bildung der zylindrischen Halteöffnung 30 enthält. Oberhalb des Außengewindeabschnittes G der Betätigungsschraube 14 weist diese einen Außenkonus $K_a$ auf. Dieser Außenkonus $K_a$ ist etwa in axialer Höhe der zylindrischen Halteöffnung 30 angeordnet. Wenn nun die Betätigungsschraube 14 angezogen wird, legt sich der Außenkonus $K_a$ zunehmend an die Außenfläche der Fixateurstange 11 an, wodurch einerseits die Außenfläche der Fixateurstange 11 gegen die Klemmbackenfläche 13 des Schlitzrohrbereichs 27 gezogen wird. Andererseits wird der Außenkonus $K_a$ selbst - und damit die Laibungsfläche L im Sinne einer Verengung - elastisch deformiert. Hierbei bleibt anzumerken, daß die Bauteile 14 sowie die Klemmschellenhalterung 12 b (ebenso wie analoge Bauteile der voraufgegangenen und nachstehend gezeigten Figuren) aus einem entropieelastisch verformbaren Kunststoff, und zwar als Spritzgußteile, hergestellt sind. Vorzuziehen ist ein Kunststoff unter Verwendung von Faserteilen, insbesondere von Kohlenstoff- oder Glasfasern.

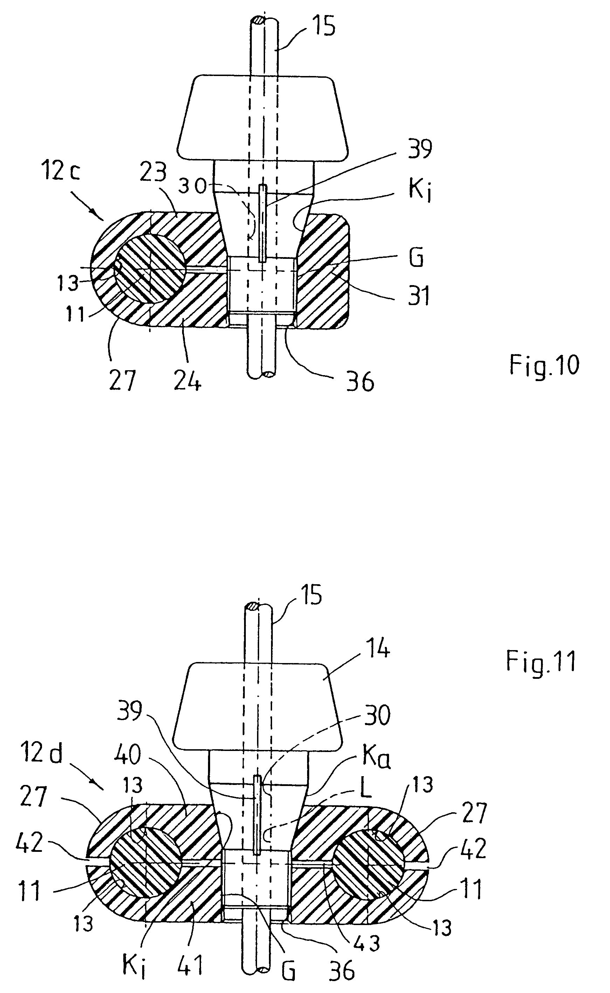

Bei den Ausführungsformen gemäß den Fig. 10-12 mit den Halterungen 12 c, 12 d, 12 e und 12 f ist die Betätigungsschraube 14 im wesentlichen identisch ausgebildet. Und zwar ist der Außenkonus $K_a$ der Betätigungsschraube 14 mit mindestens zwei Klemmschlitzen 39 versehen, welche eine Verengung der zylindrischen Halteöffnung 30 mit der Laibung L beim Anzug der Betätigungsschraube 14 erleichtern sollen. Als Widerlagerfläche bzw. Anschlag für den Außenkonus $K_a$ weist jeweils der obere Klemmschenkel 23 bzw. 40 eine dem Außenkonus $K_a$ korrelierende konische Öffnung $K_i$ (Innenkonus) auf. Der jeweils untere Klemmschenkel 24 bzw. 41 hingegen ist zur Aufnahme des Außengewindes G mit dem Innengewinde 36 versehen. Hierdurch können die Klemmschenkel 23, 24; 40, 41 (mit ihnen die Klemmbackenflächen 13) zugleich mit den zylindrischen Halteöffnungen zugestellt werden.

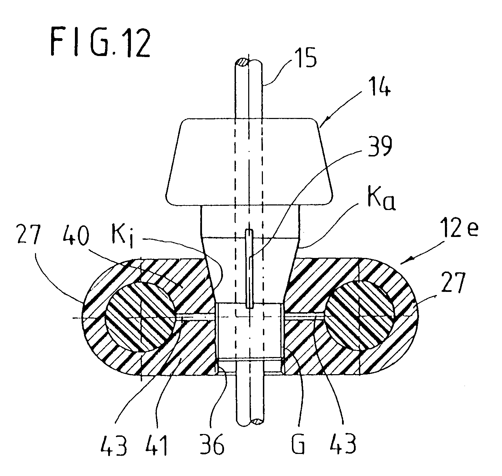

Unterschiede ergeben sich bei den Ausführungsformen gemäß den Fig. 11-12 nur insoweit, als die Fig. 11 und 12 jeweils eine Doppelschellenanordnung zur Aufnahme von zwei Fixateurstangen 11 darstellen, während Fig. 10 nur eine Einfachschelle 12 d zeigt. Die Ausführungsform gemäß Fig. 12 besitzt ungeteilte Schlitzrohrbereiche 27. Die Ausführungsform gemäß Fig. 11 weist hingegen durch Axialschlitze 42 längsgeteilte Schlitzrohrbereiche 27 auf, so daß sich in diesem Falle zwei etwa schalenförmige Klemmbauteile mit den Klemmschenkeln 40, 41 und den geteilten Schlitzrohrbereichen 27 ergeben.

Beim Ausführungsbeispiel gemäß Fig. 10 ist als Halterung nur die Einfachschelle 12 d mit einem ungeteilten Schlitzrohrbereich 27 und einer durchgehenden Werkstoffbrücke 31 zwischen den beiden Klemmschenkeln 23, 24 vorgesehen.

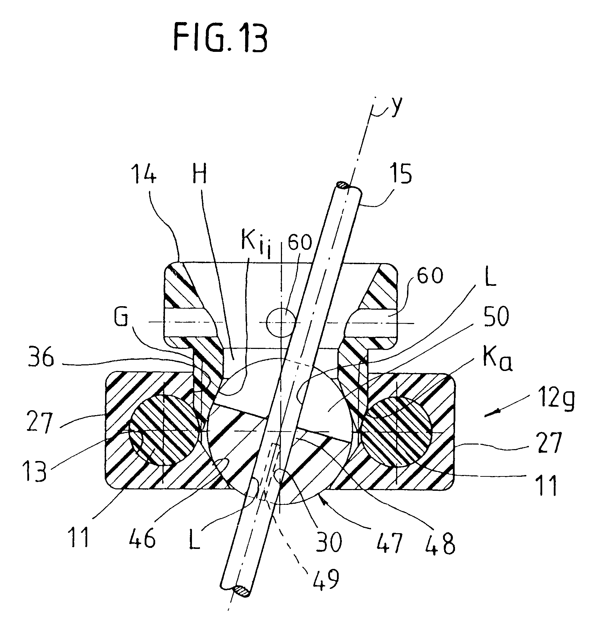

Die Ausführungsform gemäß Fig. 13 mit dem Halterungsbauteil 12 g bildet eine Abwandlung der Ausführungsform gemäß Fig. 12, jedoch mit dem Vorteil einer raumgelenkigen Orientierung und Arretierung der zylindrischen Halteöffnung 30 mit ihrer Laibung L. Zugleich wird der Vorteil einer direkten Verklemmung der beiden Fixateurstangen 11 durch die Betätigungsschraube 14 erzielt. Und zwar ist bei der Halterung 12 g eine halterungsseitige kreisringartige Kugelpfanne 46 vorgesehen, in welcher eine Klemmkugel 47 raumgelenkig gelagert ist. Die aus einem geeigneten Kunststoff bestehende Klemmkugel 47 weist zwischen einer festen Werkstoffbrücke 48 einen radial verlaufenden unteren Schlitz 49 und einen hierzu um 90° umfangswinkelversetzten radialen oberen Schlitz 50 auf. Die Klemmschlitze 49, 50 sollen die Zustellbarkeit im Bereich der Laibungsfläche L der zylindrischen Halteöffnung 30 erleichtern. Diese Zustellung erfolgt beim Ausführungsbeispiel gemäß Fig. 13 wie folgt:

Die mit dem Axialhohlraum H versehene Betätigungsschraube 14 weist an ihrem freien Ende einen Innenkonus $K_{li}$ auf, welcher außen gegen die Klemmkugel 47 drückt, während die Betätigungsschraube 14 angezogen wird. Am freien Ende des Außengewindes G der Betätigungsschraube 14 befindet sich wiederum ein Außenkonus $K_a$, welcher wiederum gegen die Außenfläche der Fixateurstangen 11 drückt. Auf diese Weise können beim Ausführungsbeispiel gemäß Fig. 13 - auch im Sinne einer Vorarretierung - eine Richtungsfixierung der Achse y, eine Verklemmung der Knochenschraube 15 innerhalb der zylindrischen Halteöffnung 30 als auch eine Arretierung der Fixateurstangen 11 innerhalb der Klemmbackenflächen 13 erfolgen.

Der Schraubenkopf der Betätigungsschraube 14 ist mit Einstecköffnungen 60 für ein Steckwerkzeug versehen.

In den Fig. 14 und 15 sind die miteinander baulich verwandten Halterungen $12_h$ und $12_i$ dargestellt.

Auch bei den Ausführungsformen gemäß den Fig. 14 und 15 ist die Schraubenachse y raumgelenkig orientier- und fixierbar. Die hier vorgesehene Klemmkugel 51 (Fig. 14) bzw. Klemmhalbkugel 52 (Fig. 15) ist als ein gesondertes Gewindeteil ausgebildet. Die gesonderten Gewindeteile 51, 52 sind mit dem Innengewinde 36 zur Aufnahme des Außengewindeteils G der Betätigungsschraube 14 versehen. Damit eine Verengung der die zylindrische Halteöffnung 30 mit der Laibung L aufnehmenden Betätigungsschraube 14 erfolgen kann, weist das Gewindeteil G im axialen Längenbereich der Laibung L jeweils einen Klemmschlitz 39 auf. Für den Fall, daß größere Klemmkräfte aufgebracht werden müssen ist es zweckmäßig, die Kugel 51 vorzusehen, während für geringere Klemmkräfte (geringe axiale Länge des Gewindes 36) eine Halbkugel 52 genügt.

Zur Aufnahme von der Kugel 51 bzw. der Halbkugel 52 ist jeder Klemmschenkel 41 mit einer ringförmigen Kugelpfanne 62 versehen.

In den Fig. 14 und 15 ist auch die bereits im Zusammenhang mit Fig. 1 erwähnte Unterlegscheibe 17 mit ihrer balligen Oberfläche 16 näher dargestellt. Die Unterlegscheibe 17 besitzt eine kreiszylindrische Grundform und ist drehbar in einer kreiszylindrischen Ausnehmung 53 angeordnet. Zur Schwenkbarkeit des Schaftes der Schraube 14 ist das Langloch (Schwenköffnung) 18 vorgesehen. Die ballige Oberfläche 16 bildet demnach das Widerlager für die Unterseite 54 des Schraubkopfes 19. Auch bei den Ausführungsformen gemäß den Fig. 14 und 15 ist eine kombinierte Arretierung auch im Sinne einer Vorarretierung sowohl der zylindrischen Halteöffnung 30, des Gewindeteile 51 bzw. 52 und der nicht dargestellten Fixateurstangen 11 innerhalb der Klemmbackenflächen 13 möglich.

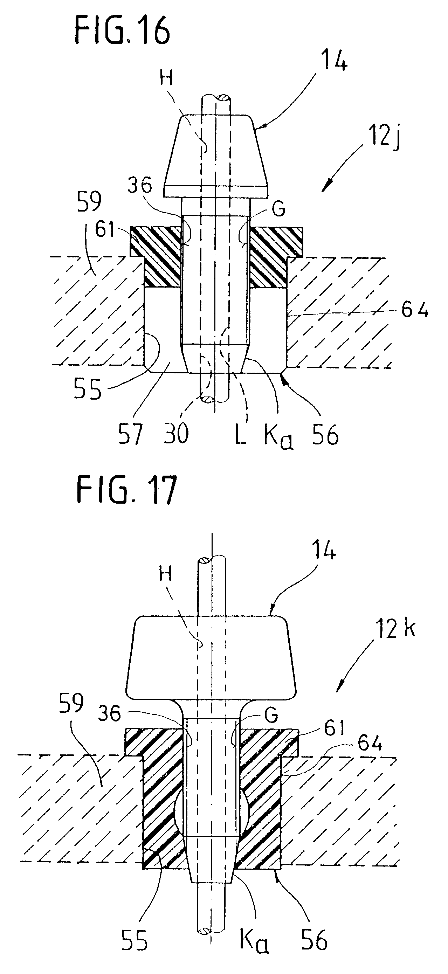

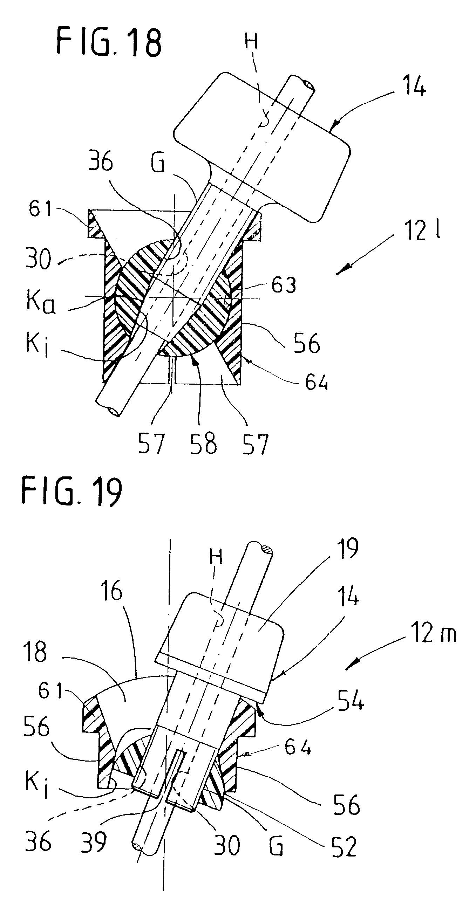

Fig. 16-19 mit den Halterungen 12 j-12 m zeigen mit Anlageflanschen 61 versehene stopfenartige Einsätze für je eine Aufnahmeöffnung 55 (in den Fig. 18 und 19 nicht dargestellt) eines etwa platten- bzw. wabenartigen Überbrückungsbauteils 59 aus für Röntgenstrahlen transparentem geeigneten Kunststoff.

Jeder stopfenartipe Einsatz 12 j-12 m mit Innengewinde 36 bildet Spreizkörper 56 mit äußeren Klemmbackenflächen 64, welche sich, gegebenenfalls erleichtert durch Klemmschlitze 57, beim Anzug der Betätigungsschraube 14 gegen die Wandung der Aufnahmeöffnung 55 andrücken. Hierzu ist die Betätigungsschraube 14 gemäß Fig. 16 außer mit dem Gewindeabschnitt G auch mit einem Außenkonus $K_a$ versehen, welcher zudem eine Deformation der zylindrischen Halteöffnung 30 mit ihrer Laibung L bewirkt. Das gleiche Prinzip wird beim Ausführungsbeispiel gemäß Fig. 17 verfolgt.

Gemäß Fig. 18 ist ein walzenförmiges Gewindeteil 58 mit einem Innengewinde 36 für das Außengewindeteil G der Betätigungsschraube 14 innerhalb einer kreiszylindrischen Lagerpfanne 63 des Einsatzes 12 l vorgesehen. Zusätzlich weist die Walze 58 einen Innenkonus $K_i$ auf, gegen den der Außenkonus $K_a$ der Betätigungsschraube 14, sowohl die elastische Deformation der Klemmwalze 58 als auch die Verengung der zylindrischen Halteöffnung 30 sowie auch eine Spreizung der Spreizkörper 56 bewirkend, anschlägt.

Beim Ausführungsbeispiel gemäß Fig. 19 weist die Halterung 12 m als gesondertes Gewindeteil mit dem Innengewinde 36 für das Außengewinde G der Betätigungsschraube 14 eine Klemmhalbkugel 52 auf, welche sich gegen die einen Innenkonus $K_i$ bildende Innenfläche des ein geschlossenes Bauteil bildenden elastisch deformierbaren Spreizkörpers 56 anlegen kann. Analog zu den Ausführungsformen der Fig. 14 und 15 weist der obere Bereich des den Spreizkörper 56 tragenden Bauteils eine langlochartige Schwenköffnung 18 auf, die von einer balligen Oberfläche 16 als Widerlager für die Unterseite 54 des Schraubkopfes 19 umgeben ist. Auch das Gewindeteil G ist im axialen Längenbereich der zylindrischen Halteöffnung 30 mit einem Klemmschlitz 39 versehen.

Allen stopfenartigen Ausführungsformen gemäß den Fig. 16-19 ist gemeinsam, daß die beweglichkeit der Bauteile 58, 59 durch die Drehbarkeit der Einsätze 12 l, 12 m innerhalb der zylindrischen Aufnahme 55 des waben- bzw. plattenförmigen Überbrückungsbauteils 59 zu einer Raumbeweglichkeit ergänzt wird.

## Patentansprüche

1. Fixateur externe (10) zur Osteosynthese, mit mindestens einem Überbrückungsbauteil 11; 59, an welchem mittels Klemmbackenflächen (13; 64) mehrere Halterungen (12; 12 a-12 e; 12 g-12 m) schraubverklemmbar sind, welche wiederum zylindrische Halteöffnungen (30) aufweisen, die mittels Betätigungsschrauben (14) zustellbar sind und hierbei als Klemmaufnahmen für je einen Knochennagel bzw. für je eine Knochenschraube (15) dienen, dadurch gekennzeichnet, daß Halterungen (12; 12 a-12 e; 12 g - 12 m) von je einer durchgehenden Laibungsfläche (L) umgrenzte zylindrische Halteöffnungen (30) aufweisen, welche bei Anzug der Betätigungsschrauben (14) gemeinsam mit benachbarten halterungsseitigen Klemmbackenflächen (13; 64) zustellbar sind und welche jeweils von einer einen Knochenbohrer mit Bewegungsspiel aufnehmenden Bohrer bzw. Schraubenführung größerer Weite bis zur Klemmaufnahme geringerer Weite für den Knochennagel bzw. für die Knochenschraube (15) elastisch oder plastisch verformbar sind.

2. Fixateur nach Anspruch 1, dadurch gekennzeichnet, daß die zylindrischen Halteöffnungen (30) bei Anzug der Betätigungsschrauben (14) durch elastische oder plastische Werkstoffverformung bezüglich des Überbrückungsbauteils (11; 59) zunächst im Sinne einer Vorarretierung, zustellbar und jeweils mit einem zusätzlichen Zustellhub von einer Bohrer- bzw. Schraubenführung bis zu ihrer Verklemmungs-Endlage mit dem Knochennagel bzw. mit der Knochenschraube (15) verformbar sind.

3. Fixateur nach einem der Ansprüche 1 bis 2, dadurch gekennzeichnet, daß eine Halterung aus zwei Klemmschellen (21, 22) mit je einem zwei parallele Klemmschenkel (25, 26; 23, 24) miteinander verbindenden Schlitzrohrbereich (28; 27) besteht, wobei der Schlitzrohrbereich (27) der einen Klemmschelle (22) die Klemm-

EP 0 240 034 B1

backenfläche (13) für das Überbrückungsbauteil (11) und der Schlitzrohrbereich (28) der anderen Klemmschelle (21) die zylindrische Halteöffnung (30) bildet, und wobei beide Klemmschellen (21; 22) mit ihren von einer gemeinsamen Betätigungsschraube (14) durchsetzten Klemmschenkeln (23-26) verspannbar sind.

4. Fixateur nach Anspruch 3, *dadurch gekennzeichnet*, daß von vier aufeinanderfolgenden Klemmschenkeln (25, 26; 23, 24) der beiden Klemmschellen (21; 22) drei Klemmschenkel (25, 26; 23) je eine glatte Durchgangsbohrung (33, 34; 35) und ein Klemmschenkel (24) eine Innengewindebohrung (36) für die gemeinsame Betätigungsschraube (14) aufweisen.

5. Fixateur nach einem der Ansprüche 1 bis 4, *dadurch gekennzeichnet*, daß die Betätigungsschraube (14) koaxial zu ihrer Schraubenlängsachse (y) von einem Axialhohlraum (H) durchsetzt ist, welcher die die Laibungsfläche (L) zur Bildung der Bohr- bzw. Schraublehre bzw. der Klemmaufnahme aufweisende zylindrische Halteöffnung (30) enthält und daß mittels derselben Betätigungsschraube (14) die Klemmbackenflächen (13; 64) zur Aufnahme des Überbrückungsbauteils (11; 59) aktivierbar sind.

6. Fixateur nach Anspruch 5, *dadurch gekennzeichnet*, daß der Axialhohlraum (H) selbst der Betätigungsschraube (14) die Laibungsfläche (L) der zylindrischen Halteöffnung (30) bildet.

7. Fixateur nach Anspruch 6, *dadurch gekennzeichnet*, daß die Betätigungsschraube (14) in ihrem gewindelosen oder mit Gewinde (bei G) versehenen Bereich im Axialbereich der zylindrischen Halteöffnung (30) mit einem sich in Einschraubrichtung verjüngenden, die zylindrische Halteöffnung (30) umfassenden Konus ($K_a$) versehen ist, welcher beim Anziehen der Betätigungsschraube (14) die zylindrische Halteöffnung (30) verengt.

8. Fixateur nach Anspruch 7, dadurch *gekennzeichnet*, daß der Konus ein Außenkonus ($K_a$) ist, welcher beim Anzug der Betätigungsschraube (14) gegen einen Anschlag (11; $K_i$) läuft.

9. Fixateur nach Anspruch 8, *dadurch gekennzeichnet*, daß der Anschlag ($K_i$) von der Halterung (12 c-12 m) gebildet ist.

10. Fixateur nach Anspruch 7 oder 8, *dadurch gekennzeichnet*, daß der Anschlag vom Überbrückungsbauteil (11) gebildet ist, welches der Außenkonus ($K_a$) beim Anzug der Betätigungsschraube (14) gegen die halterungsseitigen Klemmbackenflächen (13) drückt.

11. Fixateur nach einem der vorangehenden Ansprüche, *dadurch gekennzeichnet*, daß die Halterung aus nur einer Klemmschelle (12 b-12 e ; 12 g-12 i) besteht, deren beide einander parallele Klemmschenkel (23, 24; 40, 41) endseitig von einem Klemmbackenbereich oder von zwei Klemmbackenbereichen miteinander verbunden sind.

12. Fixateur nach Anspruch 11, *dadurch gekennzeichnet*, daß die Betätigungsschraube (14) den einen Klemmschenkel (23; 40) mit einem glatten Schaft in einer glatten Durchgangsbohrung und den anderen Klemmschenkel (24; 41) mit ihrem Gewindeschaft (G) in einer Gewindebohrung (36) durchsetzt.

13. Fixateur nach einem der Ansprüche 10 bis 12, *dadurch gekennzeichnet*, daß zwischen glattem Schaft und Gewindeschaft (G) der das Überbrückungsbauteil (11) verklemmende Außenkonus ($K_a$) vorgesehen ist.

14. Fixateur nach einem der Ansprüche 11 bis 13, *dadurch gekennzeichnet*, daß jeder Klemmbackenbereich (bei 13) aus einem zu den beiden Klemmschenkeln (23, 24; 40, 41) offenen Schlitzrohrbereich (27) besteht.

15. Fixateur nach einem der vorangehenden Ansprüche, *dadurch gekennzeichnet*, daß der die Laibungsfläche (L) der zylindrischen Halteöffnung (30) umfassende Außenkonus ($K_a$) der Betätigungsschraube (14) von mindestens einem Klemmschlitz (39) durchsetzt ist.

16. Fixateur nach einem der vorangehenden Ansprüche, *dadurch gekennzeichnet*, daß die Halterung (12 j-12 m) zur Aufnahme der mit dem Axialhohlraum (H) versehenen Betätigungsschraube (14) einen gegebenenfalls mit einem Anlageflansch (61) versehenen stopfenartigen Einsatz für eine Aufnahmeöffnung (55) des Überbrückungsbauteils (59) bildet, wobei ggf. der Einsatz, von einer Stirnseite ausgehend, über einen axialen Teilhöhenbereich geschlitzt ist und Spreizkörper (56) bei Anzug der Betätigungsschraube (14) von deren Außenkonus ($K_a$) gegen die Innenfläche der Aufnahmeöffnung (55) gedrückt werden.

17. Fixateur nach einem der vorangehenden Ansprüche, *dadurch gekennzeichnet*, daß in einer halterungsseitigen kreisringartigen Kugelpfanne (46) eine Klemmkugel (47) raumgelenkig gelagert ist, welche als zylindrische Halteöffnung (30) eine mit Klemmschlitzen (49, 50) versehene radiale Durchgangsöffnung aufweist, wobei gegen die der Kugelpfanne (46) abgewandten Seite der Klemmkugel (47) ein am freien Ende des Außengewindes (G) der Betätigungsschraube (14) vorgesehener, sich entgegen der Einschraubrichtung verjüngender Innenkonus ($K_{ii}$) drückt.

18. Fixateur nach einem der vorangehenden Ansprüche, *dadurch gekennzeichnet*, daß zusätzlich zum Innenkonus ($K_{ii}$) endseitig der Betätigungsschraube (14) ein sich in deren Einschraubrichtung verjüngender, das Überbrückungsbauteil (11) gegen die halterungsseitigen Klemmbackenflächen (13) pressender Außenkonus ($K_a$) vorgesehen ist.

19. Fixateur nach einem der vorangehenden Ansprüche, *dadurch gekennzeichnet*, daß die mit dem Axialhohlraum (H) versehene Betätigungsschraube (14) in ein gesondertes Gewindeteil (51; 52; 58) eingreift, welches gelenkig bzw. raumgelenkig in der Halterung (12 g-12 i; 12 l; 12 m) gelagert ist.

20. Fixateur nach Anspruch 19, dadurch gekennzeichnet, daß das gesonderte Gewindeteil eine mit einer zentralen Innengewindebohrung (36) versehene Walze (58), Kugel (51) oder Halbkugel (52) ist, welche in einer halterungsseitigen kreiszylindrischen oder kugeligen Lagerpfanne (62, 63) aufgenommen ist.

21. Fixateur nach Anspruch 19 oder nach Anspruch 20, dadurch gekennzeichnet, daß die kugelige Lagerpfanne (62) zur Aufnahme des kugeligen Gewindeteils (51; 52) in einem Klemmschenkel (41) einer Klemmschelle (12 h; 12 i) angeordnet ist, während der andere Klemmschenkel (40) zur Abstützung des Schraubkopfes (54) der Betätigungsschraube (14) eine ballige Oberfläche (16) aufweist, die eine Schwenköffnung (18) umgibt.

22. Fixateur nach Anspruch 21, dadurch gekennzeichnet, daß die ballige Oberfläche (16) von einer in eine kreiszylindrische Ausnehmung (53) eines Klemmschenkels (40) drehbar aufgenommenen Unterlegscheibe (17) gebildet ist, welche als Schwenköffnung ein Langloch (18) aufweist.

23. Fixateur nach einem der vorangehenden Ansprüche, dadurch gekennzeichnet, daß bei einer nur einen Schlitzrohrbereich (27; 28) für die Klemmbackenflächen (13) bzw. für die zylindrische Halteöffnung (30) aufweisenden Klemmschelle (22; 21; 12 c) beide Klemmschenkel (23, 24; 25, 26) endseitig über eine Werkstoffbrücke (31; 32) einstückig miteinander verbunden sind.

24. Fixateur nach einem der vorangehenden Ansprüche, dadurch gekennzeichnet, daß die Halterungen (12, 12 a-12 e; 12 g-12 m) einschließlich der Betätigungsschrauben (14) und Gewindeaufnahmen (51; 52; 58) Spritzgußteile, vornehmlich Kunststoff-Spritzgußteile sind.

25. Fixateur nach Anspruch 24, dadurch gekennzeichnet, daß die Kunststoffspritzgußteile faserverstärkt sind.

26. Fixateur nach einem der vorangehenden Ansprüche, dadurch gekennzeichnet, daß zwischen die Klemmbackenflächen (13; 64) und/oder in die zylindrischen Halteöffnungen (30) umfangsveränderliche Paßbuchsen bzw. Paßringe, wie z.B. längsgeschlitzte oder flexible Paßbuchsen (29) bzw. Paßringe, einsetzbar sind.

27. Fixateur nach einem der vorangehenden Ansprüche, dadurch gekennzeichnet, daß gegeneinander verspannbare relativbewegliche Flächen von Halterungen bzw. Betätigungsschrauben (14) formschlüssig ineinandergreifen, z.B. mit einer rauhen Oberfläche oder mit einer Plan-Kerb-Verzahnung (38), sogenannte "Hirth-Verzahnung", versehen sind.

## Claims

1. An external fastener (10) for osteosynthesis, comprising at least one bridging component (11; 59), to which a plurality of securing elements (12; 12a-12e; 12g-12m) can be screw-clamped using clamping jaw surfaces (13; 64), which securing elements in turn comprise cylindrical retaining openings (30), which are closable by means of operating screws (14) and in this respect act as clamping recesses for a bone pin or bone screw (15) in each case, characterised in that the securing elements (12; 12a-12e; 12g-12m) comprise cylindrical retaining surfaces (30), which are enclosed by a continuous soffit surface (L) in each case, are closable together with adjacent clamping jaw surfaces (13; 64) on the securing elements when the operating screws (14) are tightened and are elastically or plastically deformable from a drill or screw guide of larger width receiving a bone drill with clearance of movement to a clamping recess of reduced width for the bone pin or bone screw (15).

2. A fastener according to claim 1, characterised in that, when the operating screw (14) is tightened, the cylindrical retaining openings (30) are closable relative to the bridging component (11; 59) firstly with a view towards pre-locking by way of an elastic or plastic material deformation and with an additional closing stroke are closable from a drill or screw guide into their final clamping position with the bone pin or bone screw (15).

3. A fastener according to one of claims 1 to 2, characterised in that a securing element comprises two clamping collars (21, 22) each with a slotted tube region (28; 27) connecting two parallel clamping limbs (25, 26; 23, 24) with one another, the slotted tube region (27) of one clamping collar (22) forming the clamping jaw surface (13) for the bridging component (11) and the slotted tube region (27) of the other clamping collar (21) forming the cylindrical retaining opening (30), and both clamping collars (21; 22) being clampable with their clamping limbs (23-26) penetrated by a common operating screw (14).

4. A fastener according to claim 3, characterised in that, of the four successive clamping limbs (25, 26; 23, 24) of the two clamping collars (21; 22), three clamping limbs (25, 26; 23) each comprise a smooth through bore (33, 34; 35) and one clamping limb (24) comprises an internal threaded bore (36) for the common operating screw (14).

5. A fastener according to one of claims 1 to 4, characterised in that the operating screw (14) is penetrated coaxial to its screw longitudinal axis (y) by an axial cavity (H), which contains the cylindrical opening (30) comprising the soffit surface (L) for forming the drill or screw guide or the clamping recess, and the clamping jaw surfaces (13; 64) for receiving the bridging component (11; 59) can be activated by the same operating screw (14).

6. A fastener according to claim 5, characterised in that the axial cavity (H) per se of the operating screw (14) forms the soffit surface (L) of the cylindrical retaining opening (30).

7. A fastener according to claim 6, characterised in that, in its threadless region or region provided with a thread (at G), the operating screw (14) is provided in the axial region of the cylindrical retaining opening (30) with a cone ($K_a$), which encloses the cylindrical retaining opening (30), narrows in the screwing-in direction and reduces the size of the cylindrical retaining opening (30) when the operating screw (14) is tightened.

8. A fastener according to claim 7, characterised in that the cone is an external cone (Ka), which runs against an abutment (11; $K_i$) when the operating screw (14) is tightened.

9. A fastener according to claim 8, characterised in that the abutment (Ki) is formed by the securing element (12c-12m).

10. A fastener according to claim 7 or 8, characterised in that the abutment is formed by the bridging component (11), which presses the external cone (Ka) against the clamping jaw surfaces (13) on the the securing element when the operating screw (14) is tightened.

11. A fastener according to one of the preceding claims, characterised in that the securing element is formed by a single clamping collar (12b-12e; 12g-12i), whose two parallel clamping limbs (23, 24; 40, 41) are connected with one another at their end faces by a clamping jaw region or two clamping jaw regions.

12. A fastener according to claim 11, characterised in that the operating screw (14) penetrates one clamping limb (23; 40) with a smooth shaft in a smooth through bore and the other clamping limb (24; 41) with its threaded shaft (G) in a threaded bore (36).

13. A fastener according to one of claims 10 to 12, characterised in that the external cone (Ka) clamping the bridging component (11) is provided between the smooth and the threaded shaft (G).

14. A fastener according to one of claims 11 to 13, characterised in that each clamping jaw region (at 13) is formed by a slotted tube region (27) open towards both clamping limbs (23, 24; 40, 41).

15. A fastener according to one of the preceding claims, characterised in that the external cone (Ka) of the operating screw (14) enclosing the soffit surface (L) of the cylindrical retaining opening (30) is penetrated by at least one clamping slot (39).

16. A fastener according to one of the preceding claims, characterised in that, for receiving the operating screw (14) provided with the axial cavity (H), the securing element (12j-12m) forms a plug-like insert optionally provided with an abutment flange (61) for a receiving opening (55) of the bridging component (59), the insert optionally being slotted over a partial axial height region, proceeding from an end face, and expanding elements (56) are pressed when the operating screw (14) is tightened against the internal surface of the receiving opening (55) by the external cone (Ka) of said screw.

17. A fastener according to one of the preceding claims, characterised in that a clamping ball (47) is mounted so as to swivel triaxially in a circular ring-shaped ball cup (46) on the securing element, the clamping ball (47) comprising as a cylindrical retaining opening (30) a radial through opening provided with clamping slots (49, 50), an internal cone (Kii) provided at the free end of the external thread (G) of the operating screw (14) and narrowing in the opposite direction to the screwing-in direction pressing against the side of the clamping ball (47) facing away from the ball cup (46).

18. A fastener according to one of the preceding claims, characterised in that, in addition to the internal cone (Kii) at the end of the operating screw (14), an external cone (Ka) is also provided, which narrows in the screwing-in direction of said screw and presses the bridging component (11) against the clamping jaw surfaces (13) on the securing element.

19. A fastener according to one of the preceding claims, characterised in that the operating screw (14) provided with the axial cavity (H) engages in a separate threaded element (51; 52; 58), which is mounted in the securing element (12g-12i; 12l; 12m) in articulated or triaxially pivotal fashion.

20. A fastener according to claim 19, characterised in that the separate threaded element is a roller (58), ball (51) or hemisphere (52) provided with a central internal threaded bore (36) and accommodated in a circular cylindrical or spherical bearing cup (62, 63) in the securing element.

21. A fastener according to claim 19 or 20, characterised in that, for receiving the spherical threaded element (51; 52), the spherical bearing cup (62) is arranged in one clamping limb (41) of a clamping collar (12h; 12i), whilst the other clamping limb (40) comprises a spherical surface (16) surrounding a pivot opening (18) for supporting the screw head (54) of the operating screw (14).

22. A fastener according to claim 21, characterised in that the spherical surface (16) is formed by a washer (17), which is rotatably accommodated in a circular cylindrical recess (53) of a clamping limb (40) and comprises a slot (18) as a pivot opening.

23. A fastener according to one of the preceding claims, characterised in that, in the case of a clamping collar (22; 21; 12c) comprising only one slotted tube region (27; 28) for the clamping jaw surfaces (13) or the cylindrical retaining opening (30), both clamping limbs (23, 24; 25, 26) are integrally connected with one another at their ends via a material bridge (31; 32).

24. A fastener according to one of the preceding claims, characterised in that the securing elements (12; 12a-12e; 12g-12m) including the operating screws (14) and threaded receiving elements (51; 52; 58) are injection molded parts, preferably plastics material injection molded parts.

25. A fastener according to claim 24, characterised in that the plastics material injection molded parts are fibre-reinforced.

26. A fastener according to one of the preceding claims, characterised in that circumferentially-variable fitting bushings or fitting rings, such as longitudinally slotted or flexible fitting bushings (29) or fitting rings, can be inserted between the clamping jaw surfaces (13; 64) and/or in the cylindrical retaining openings (30).

27. A fastener according to one of the preceding claims, characterised in that surfaces of securing elements or operating screws (14) movable relative to one another and clampable against one another, engage in one another in a positive-locking manner, e.g. are provided with a rough surface or with a face-groove-toothing (38), or so-called "Hirth toothing".

## Revendications

1. Fixateur externe (10) pour ostésynthèse, comportant au moins une pièce de pontage (11; 59), sur laquelle plusieurs dispositifs de fixation (12; 12a à 12e; 12g à 12m) peuvent être bloqués par vissage, à l'aide de mâchoires de serrage (13; 64), ces dispositifs de fixation présentant des ouvertures de positionnement cylindriques (30), qui peuvent être resserrées à l'aide de vis de réglage (14) et dont chacune accueille un clou pour fractures ou une broche (15), caractérisé par le fait que les dispositifs de fixation (12; 12a à 12e; 12g à 12m) présentent des ouvertures de positionnement cylindriques (30), dont chacune est délimitée par une surface interne courbe, continue (L), lesquelles peuvent être resserrées à l'aide des vis de réglage (14), en commun avec leurs mâchoires de serrage (13; 64) avoisinantes, et soumises ainsi à une déformation élastique ou plastique, pouvant les faire passer d'une dimension élevée, pour l'introduction, avec un certain jeu, d'un trépan ou un d'un taraud, à une dimension réduite, pour le blocage du clou pour fractures ou de la broche (15).

2. Fixateur selon la revendication 1, caractérisé par le fait que, lors du serrage des vis de réglage (14), les ouvertures de positionnement cylindriques (30) pour l'élément de pontage (11; 59) peuvent être resserrées par déformation élastique ou plastique, tout d'abord au sens d'un blocage préalable, le resserrement étant réglé ensuite, par tours de vis supplémentaires, en fonction de la taille d'un trépan ou d'un taraud, jusqu'au blocage final du clou pour fractures ou de la broche (15).

3. Fixateur selon l'un des revendications 1 ou 2, caractérisé par le fait qu'une fixation comporte deux brides de serrage (21, 22), dont chacune présente une zone tubulaire fendue (28; 27), reliant deux branches de serrage parallèles (25, 26; 23, 24), la zone tubulaire fendue (27) de l'une des brides de serrage (22) formant la mâchoire de serrage (13), destinée à recevoir la tige (11), tandis que la zone tubulaire fendue (28) de l'autre bride de serrage (21) forme l'ouverture de positionnement cylindrique (30), les deux brides de serrage (21; 22) pouvant être déformées à leurs branches de serrage (23 à 26), traversées par une vis de réglage (14) commune.

4. Fixateur selon la revendication 3, caractérisé par le fait que des quatre branches de serrage successives (25, 26; 23, 24) des deux brides de serrage (21; 22), trois (25, 26; 23) présentent, chacune, un orifice de passage lisse (33, 34; 35), tandis que l'autre branche de serrage (24) est pourvue d'un filetage intérieur (36) pour la vis de réglage (14) commune.

5. Fixateur selon l'une des revendication 1 à 4, caractérisé par le fait que la vis de réglage (14) est traversée, coaxialement par rapport à son axe longitudinal (y), par une cavité (H), qui comprend l'ouverture de positionnement cylindrique (30), avec sa surface intérieure courbe (L) devant former le gabarit du trépan, du taraud ou de l'ouverture de blocage, et que les mâchoires de serrage (13) peuvent être actionnées à l'aide de la même vis de réglage (14) pour accueillir l'élément de pontage (11; 59).

6. Fixateur selon la revendication 5, caractérisé par le fait que la cavité axiale (H) de la vis de réglage (14) forme, elle-même, la surface intérieure (L) de l'ouverture de positionnement cylindrique (30).

7. Fixateur selon la revendication 7, caractérisé par le fait que la tige de la vis de réglage (14) filetée (G) ou non, est équipée, dans la zone axiale de la l'ouverture de positionnement cylindrique (30), d'un cône ($K_a$), comprenant l'ouverture de positionnement cylindrique (30) et s'amincissant dans le sens de vissage, lequel cône $K_a$ resserre l'ouverture de positionnement cylindrique (30) lors du serrage de la vis de réglage (14).

8. Fixateur selon la revendication 7, caractérisé par le fait que le cône est un cône externe $K_a$, qui heurte une butée (11; $k_i$) lors du serrage de la vis de réglage (14).

9. Fixateur selon la revendication 8, caractérisé par le fait que la butée ($K_i$) est formée par le dispositif de fixation (12c à 12m).

10. Fixateur selon la revendication 7 ou 8, caractérisé par le fait que la butée est formée par l'élément de pontage (11), qui est pressé contre les mâchoires de serrage (13) du dispositif de fixation, par le cône externe ($k_a$), lors du serrage de la vis de réglage (14).

11. Fixateur selon l'une des revendications précédentes, caractérisé par le fait que le dispositif de fixation consiste en une seule bride de serrage (12b à 12e; 12g à 12i), dont les deux branches de serrage parallèles (23, 24; 40, 41) sont reliées par une mâchoire de serrage ou deux.

12. Fixateur selon la revendication 11, caractérisé par le fait que la vis de réglage (14) traverse, avec sa tige lisse, une perforation lisse de l'une des branches de serrage (23; 40) et, avec sa tige filetée (G), une perforation filetée (36) de l'autre branche de serrage (24; 41).

13. Fixateur selon l'une des revendications 10 à 12, caractérisé par le fait que le cône externe ($K_a$), bloquant l'élément de pontage (11) est prévu entre la tige lisse et la tige filetée (G).

14. Fixateur selon l'une des revendications 11 à 13, caractérisé par le fait que chaque mâchoire de serrage (13) consiste en une zone tubu-laire fendue (27), ouverte en direction des deux branches de serrage (23, 24; 40, 41).

15. Fixateur selon l'une des revendications précédentes, caractérisé par le fait que le cône externe $K_a$ de la vis de réglage (14), comprenant la surface intérieure (L) de l'ouverture de positionnement cylindrique (30), est pourvu d'au moins une fente de blocage (39).

16. Fixateur selon l'une des revendications précédentes, caractérisé par le fait que les dispositifs de fixation (12j à 12m), accueillant la vis de réglage (14) à cavité axiale (H), forment, chacun, un insert, qui bouche l'ouverture de réception (55) de la pièce de pontage (59), lequel insert est pourvu, le cas échéant, d'une bride de contact (61) et est, éventuellement fendu, à partir de la partie frontale, sur une partie de la hauteur d'une zone axiale, des écarteurs (56) étant pressés contre la surface intérieure de l' orifice de réception (55) par le cône externe ($K_a$) de la vis de réglage (14), lors du serrage de cette dernière.

17. Fixateur selon l'une des revendications précédentes, caractérisé par le fait qu'une bille de blocage (47) est logée dans une cuvette annulaire (46), qui lui assure une mobilité tridimen-sionnelle, cette bille de blocage (47) présentant, comme ouverture de positionnement cylindrique (30), un trou de passage radial, pourvu de fentes de blocage (49, 50), et qu'un cône interne ($k_{ii}$), prévu à l'extrémité libre de la tige à filetage extérieur (G) de la vis de réglage (14) et s'amincissant dans le sens opposé au sens de vissage, vient s'appuyer contre la bille de blocage (47), du côté opposé à la cuvette (46).

18. Fixateur selon l'une des revendications précédentes, caractérisé par le fait qu'en plus du cône interne ($k_{ii}$), un cône externe ($K_a$), pressant l'élément de pontage (11) contre les mâchoires de serrage (13) des dispositifs de fixation, est prévu à l'extrémité de la vis de réglage (14), dans le sens de vissage de laquelle il s'amincit.

19. Fixateur selon l'une des revendications précédentes, caractérisé par le fait que la vis de réglage (14), pourvue d'une cavité axiale (H), mord dans une pièce filetée, séparée (51; 52; 58), logée dans le dispositif de fixation (12g à 12i; 12l; 12m), lui assurant une mobilité tridimensionnelle.

20. Fixateur selon la revendication 19, caractérisé par le fait que la pièce filetée, séparée est une pièce cylindrique (58), une bille (51) ou une demi-bille (52) avec perforation centrale, pourvue d'un filetage intérieur (36) et logée dans une crapaudine (62, 63) en forme de cylindre de révolution ou sphérique.

21. Fixateur selon la revendication 19 ou 20, caractérisé par le fait que la crapaudine sphérique (62), recevant la pièce sphérique filetée (51; 52) est disposée dans une branche de serrage (41) d'une bride de serrage (12h; 12i), tandis que l'autre branche de serrage (40) présente une surface bombée (16), qui entoure une ouverture d'orientation (18) et soutient la tête (54) de la visse de réglage (14).

22. Fixateur selon la revendication 1, caractérisé par le fait que la surface bombée (16) est formée par une rondelle (17), logée, rotative, dans une cavité (53), en forme de cylindre de révolution, de l'une des branches de serrage (40) et présentant une ouverture d'orientation oblongue (18).

23. Fixateur selon l'une de revendications précédentes, caractérisé par le fait que dans l'une des brides de serrage (22; 21; 12c) ne comprenant qu'une zone tubulaire fendue (27; 28) pour les mâchoires de serrage (13) ou pour l'ouverture de positionnement cylindrique (30), les deux branches de serrage (23, 24; 25, 26) sont reliées, à leurs extré-mités, par un pont (31; 32), formant ainsi une seule pièce.

24. Fixateur selon l'une des revendications précédentes, caractérisé par le fait que les dispositifs de fixation (12; 12a à 12e; 12g à 12m), la vis de réglage (14) et les éléments filetés (51; 52; 58) sont des pièces moulées par injection, principalement en matière plastique.

25. Fixateur selon la revendication 24, caractérisé par le fait que les pièces moulées par injection en matière plastique sont renforcées par fibres.

26. Fixateur selon l'une des revendications précédentes, caractérisé par le fait que des coussinets ou des bagues d'ajustage de circonférence variable, tels que, par exemple, des fentes longitudinales ou des coussinets d'ajustage flexibles (29) ou des bagues d'ajustage flexibles peuvent être insérés entre les mâchoires de serrage (13; 64) et/ou dans les ouvertures de positionnement cylindriques (30).

27. Fixateur selon l'une des revendications précédentes, caractérisé par le fait que les surfaces des dispositifs de fixation ou de la vis de réglage (14) déformables, relativement mobiles par rapport l'une à l'autre, s'engrènent mécaniquement, étant pourvues, par exemple, d'une surface rugueuse ou d'une denture plate (38), dite "denture Hirth".

Fig. 1

Fig.2

Fig. 3

Fig.4

Fig.5

Fig.6

Fig.7

Fig. 8

Fig. 9

Fig.10

Fig.11

# FIG.12

# FIG.13

FIG.14

FIG.15

## FIG.16

## FIG. 17

# FIG. 18

# FIG. 19